(19) Europäisches Patentamt
European Patent Office
Office européen des brevets

(11) Publication number: **0 115 429**
**B1**

# (12) EUROPEAN PATENT SPECIFICATION

(45) Date of publication of patent specification: **21.01.87**

(51) Int. Cl.$^4$: **C 07 C 25/18**

(21) Application number: **84300480.5**

(22) Date of filing: **26.01.84**

## (54) Biphenyls and their use in the production of liquid crystal compounds.

(30) Priority: **26.01.83 GB 8302119**
**20.12.83 GB 8333897**

(43) Date of publication of application:
**08.08.84 Bulletin 84/32**

(45) Publication of the grant of the patent:
**21.01.87 Bulletin 87/04**

(84) Designated Contracting States:
**CH DE FR GB LI**

(58) References cited:
**GB-A-1 460 295**

(73) Proprietor: **The Secretary of State for Defence in Her Britannic Majesty's Government of the United Kingdom of Great Britain and Northern Ireland Whitehall**
**London SW1A 2HB (GB)**

(73) Proprietor: **Merck Patent Gesellschaft mit beschränkter Haftung**
**Frankfurter Strasse 250**
**D-6100 Darmstadt (DE)**

(72) Inventor: **Bishop, David Ian**
**19 Freshwater Drive**
**Hamworthy Poole Dorset (GB)**
Inventor: **Sage, Ian Charles**
**238 Freshwater Drive**
**Hamworthy Poole Dorset (GB)**
Inventor: **McDonnell, Damien Gerard**
**Flat 10 Eton Hurst Upper Chase Road**
**Malvern, Worcs. (GB)**

(74) Representative: **McCormack, Derek James**
**Procurement Executive Ministry of Defence Pats 1A(4), Room 2014 Empress State Building Lillie Road**
**London SW6 1TR (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 115 429 B1

**Description**

Liquid crystal materials which are of the type known in the art as chiral nematic materials generally consist of one or more chiral nematogenic compounds which are optically active compounds exhibiting the property or tendency of forming liquid crystal phases. These materials may be used for example in various kinds of electro-optical device employing appropriate effects, eg the so-called "phase change" effect. They may also be used in various temperature sensing applications involving the selective reflection of radiation of different wavelengths dependent on temperature. For such temperature sensing applications the selective reflection preferably occurs in the visible spectral region.

According to the present invention in a first aspect a novel fluorobiphenyl auitable for use in the preparation of chiral nematogenic compounds has a formula:

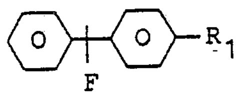

Formula I

wherein

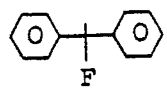

represents a 4-biphenylyl radical carrying a fluorine substituent in at least one lateral position;

$R_1$ is an optically active alkyl group (+)-$CH_3.CH_2.CH(CH_3).CH_2$ and which is a separate optically active isomer.

The symbol (+)- indicates an optically active group showing a positive optical rotation angle.

Preferably, fluorine is present in the 2 or 2′ position (the group $R_1$ being in the 4-position).

Although laterally fluorinated 4-alkylbiphenyls are known such compounds have not hitherto been suggested in a form in which the terminal alkyl group is a chiral group, ie contains an optically active centre.

According to the present invention in a second aspect there is provided the use of a compound of Formula I in the preparation of chiral nematogenic compounds suitable for use in chiral nematic liquid crystal materials.

Examples of chiral nematogenic compounds which may be prepared for the biphenyls of Formula I are the compounds of Formula II as follows:

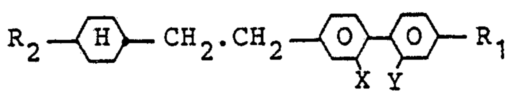

Formula II

wherein $R_2$ is n-alkyl, and X and Y are selected from H and F, at least one being F.

Liquid crystalline compounds synthesised from compounds of Formula I unexpectedly can have advantageous properties, eg can show selective reflection of light at room temperature as exemplified below.

Compounds of Formula I may be produced by the route:

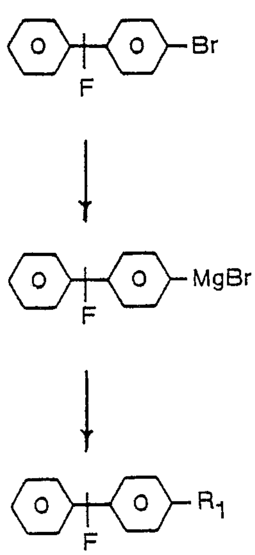

Examples of the preparation and properties of compounds for Formulae I and II given above will now be described by way of example. In the following Examples the meanings of the various symbols used are as follows:

glc = gas liquid chromatography
bp = boiling point
mp = melting point
Ch—I = chiral nematic to isotropic liquid transition temperature
S—Ch = Smectic to chiral nematic transition temperature
K—Ch = Crystalline solid to chiral nematic transition tempeature

Example 1

The preparation of (+)-1-(trans-4-n-butylcyclohexyl-2-[2′-fluoro-4′(2-methylbutyl)-4-biphenylyl]-ethane:

Step P: The preliminary preparation of (+)-2 Fluoro-4-(2-methylbutyl)-biphenyl

10 ml of a solution of a 4-bromo-2-fluorobiphenyl (90 gram) in tetrahydrofuran (90 ml) was added to magnesium turnings (9.6 gram) and tetrahydrofuran (20 ml) under nitrogen. A single crystal of iodine was added and the reaction was initiated by warming. The remainder of the solution of 4-bromo-2-fluorobiphenyl was then added dropwise over ½ hour. After heating under reflux for 1 hour, the solution of the Grignard reagent was cooled to 25°C. 10 ml of a solution of 2-methylbutylphenylsulphonate (150 gram) in tetrahydrofuran (140 ml) was then added to the reaction mixture followed by cuprous chloride (3 gram). The remainder of the phenylsulphonate solution was added over a 40 minute period. The temperature of the resulting grey/green reaction mixture rose to 45°C which was then elevated by heating to boiling for 1 hour. The reaction was allowed to cool to room temperature, then added to a 20% (by volume) hydrochloric acid solution (2.5 litres) and the product extracted with dichloromethane (2 × 500 ml). The organic layer was washed with water (2 × 500 ml) dried over anhydrous sodium sulphate (15 gram) and the solvent evaporated to give an orange liquid residue. On cooling some white solid crystallised out from the residue. After filtration the residue (88.6 gram) was fractionally distilled under vacuum (0.5 torr) to give a colourless liquid (65 gram). This was found to be 98.8% pure by glc. The bp was 120°C at 0.5 torr. The optical rotation was + 14.62° (solvent).

Step 1a2: The preparation of (+)-4-(trans-4-n-Butylcyclohexylacetyl)-2′-fluoro-4′-(2-methylbutyl)biphenyl

A mixture of (+)-2-Fluoro-4(2-methylbutyl)biphenyl (10 gram) and *trans*-4-*n*-butylcyclohexylacetyl chloride (9.4 gram) in dichloromethane (20 ml) was added over 20 minutes to a cooled (5—10°C), stirred suspension of anhydrous aluminium chloride (6.1 gram) in dichloromethane (20 ml). After the addition the reaction mixture was allowed to warm to 23°C and left stirring for 17 hours. The resulting solution was added to water (200 ml), extracted successively with petroleum spirit (bp. 60—80°: 180 ml +100 ml). The organic extract was then washed with water (200 ml), dried over anhydrous sodium sulphate (5 gram) and evaporated. The yellow crystalline residue (17.9 gram) was recrystallised from ethanol (35 ml) at 25°C to give the product (14.8 gram; 85% yield). The product had mp = 71.4—72.1°C; Virtual Ch—I, (68.5—68.8°C). It was found to be 99.5% pure by glc.

Step 1b2: The preparation of (+)-1-trans-4-n-Butylcyclohexyl)2-[2′-fluoro-4′(2-methylbutyl)-4-biphenylyl]-ethane

The ketone prepared above (14.4 gram), 99% hydrazine hydrate (14.4 ml) potassium hydroxide (7.2 gram) and digol (120 ml) were heated with stirring under a reflux condenser at 125°C for 4 hours, after which time temperature was raised to 175°C by distillation of the excess hydrazine hydrate. The mixture was then heated under reflux for 17 hours, cooled to 60°C and poured onto ice water (500 gram). The organic product was extracted with petroleum spirit (bp. 60—80°:2 × 200 ml), the extract washed with water (2 × 200 ml) and dried over anhydrous sodium sulphate. Evaporation of the solvent yielded a yellow oil (14.1 gram). This was dissolved in petroleum spirit (bp 60—80:100 ml) and adsorbed onto a column of basic alumina (50 gram) over silica gel (20 gram). Elution with petroleum spirit (320 ml) gave on evaporation of the solvent a colourless oil (11.7 gram). Crystallisation from propan-1-ol (70 ml) at −50°C gave a chiral nematic material 9.4 gram; 68% yield). This was found to be 99.8% pure by glc.

The product had the properties: S—Ch, = 6°C; Ch—I, 72°C
Helical molecular pitch = 0.23 microns.

Example 2

The preparation of 1-(trans-4-ethylcyclohexyl)-2-[2′-fluoro-4′-(2-methylbutyl)-4-biphenylyl] ethane:

Step 1a3: The preparation of 4-(trans-4-ethylcyclohexylacetyl)-2′-fluoro-4′(2-methylbutyl)biphenyl

This was carried out in a manner analogous to Step 1a2 using 4-ethylcyclohexyl acetyl chloride as starting material.

The product had the properties: Mp = 40—42°C; virtual Ch—I = (27.5°C)

Step 1b3: The preparation of 1-(trans-4-ethylcyclohexyl))-2-[2′-fluoro-4′-(2-methylbutyl)-4-biphenylyl]-ethane

This was carried out in manner analogous to Step 1b2 above.

This product has the properties: S—Ch = −11°C Ch—I = 45°C

Example 3

The preparation of 1-(trans-4-n-heptylcyclohexyl)-2-[2′-fluoro-4′-(2-methylbutyl)4-biphenylyl]ethane:

Step 1a4: The preparation of 4-(trans-4-n-heptylcyclohexylacetyl)-2′-fluoro-4′(2-methylbutyl)biphenyl

This was carried out in a manner analogous to Step 1a2 above.

The product had the properties: K—Ch = 69.8—70.3°C; Ch—I = 74.1—74.3°C

Step 1b4: The preparation of 1-(trans-4-n-heptylcyclohexyl)-2-[2′-fluoro-4′-(2-methylbutyl)-4-biphenylyl]-ethane

This was carried out in a manner analogous to Step 1b2 above.

The product had the properites: S—Ch = 52°C Ch—I = 79°C

**Claims**

1. A fluorobiphenyl suitable for use in the preparation of chiral nematographic compounds and having a formula

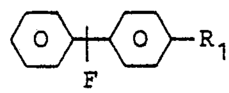

wherein

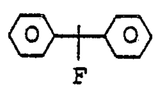

represents a 4-biphenylyl radical carrying a fluorine substituent in at least one lateral position, ie in at least one of the 2, 3, 2′ or 3′ positions, and $R_1$ is an optically active group (+)-$CH_3CH_2CH(CH_3).CH_2$ and which is a separate optically active isomer.

2. A fluorobiphenyl as claimed in claim 1 and wherein

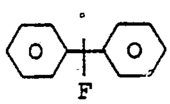 is selected from 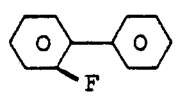 and 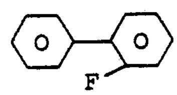

3. The use of fluorobiphenyl as claimed in claim 1 in the production of chiral nematic 4,4′-disubstituted biphenyls carrying a lateral fluorine substituent.

4. The use of fluorobiphenyl as claimed in claim 1 in the production of chiral nematic ethanes of formula:

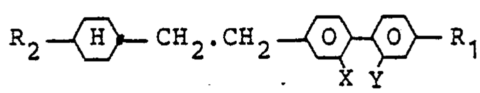

where $R_2$ is n-alkyl and X and Y are selected from H and F, at least one being F.

**Patentansprüche**

1. Fluorbiphenyl, das zur Verwendung bei der Herstellung von nematogenen Verbindungen geeignet ist und die Formel hat

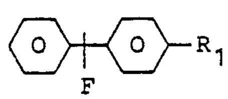

wobei

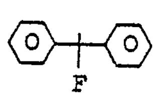

ein 4-Biphenylyl-Radikal mit einem Fluorsubstituenten in mindestens einer seitlichen Position, d.h. in mindestens eine der 2-, 3-, 2′- oder 3′-Positionen darstellt und $R_1$ eine optisch aktive Gruppe (+)-$CH_3CH_2CH(CH_3).CH_2$ ist, die ein separater optisch aktiver Isomer ist.

2. Fluorobiphenyl nach Anspruch 1, wobei

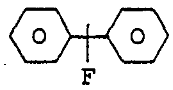 aus 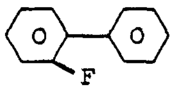 und 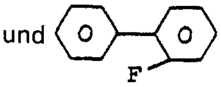

ausgewählt ist.

3. Verwendung eines Fluorobiphenyls nach Anspruch 1 bei der Herstellung von chiralen nematischen 4,4'-disubstituierten Biphenylen mit einem seitlichen Fluorsubstituenten.

4. Verwendung eines Fluorbiphenyls nach Anspruch 1 bei der Herstellung von chiralen nematischen Äthanen der Formel

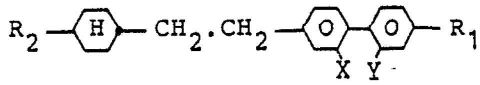

wobei $R^2$ n-Alkyl und X und Y aus H und F ausgewählt sind und mindestens eines F ist.

**Revendications**

1. Fluorobiphényle convenant à l'utilisation dans la préparation de composés chiraux nématogènes et présentant une formule

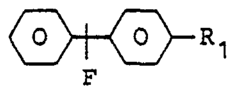

dans laquelle

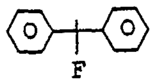

représente un radical 4-biphénylyle portant un substituant fluoré dans au moins une position latérale, c'est-à-dire dans au moins une des positions 2, 3, 2' ou 3', et $R_1$ est un groupe optiquement actif (+)-$CH_3CH_2CH(CH_3).CH_2$, et lequel est un isomère séparé optiquement actif.

2. Fluorobiphényle selon la revendication 1 et dans lequel

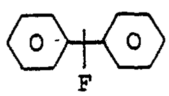 est choisi parmi 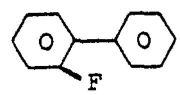 et 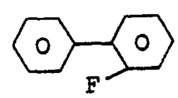

3. Utilisation d'un fluorobiphényle selon la revendication 1 dans la production de 4, 4'-biphényles disubstitués, chiraux nématiques portant un substituant fluoré latéral.

4. Utilisation d'un fluorobiphényle selon la revendication 1 dans la production d'éthanes chiraux nématiques de formule:

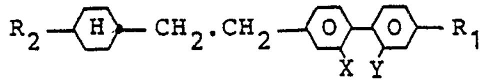

dans laquelle $R^2$ représente un n-alcoyle et X et Y sont choisis parmi H et F, l'un au moins étant F.